# EUROPEAN PATENT SPECIFICATION

(11) **EP 3 206 492 B1**
(45) Date of publication and mention of the grant of the patent: **10.04.2019**
(21) Application number: 14812306.0
(22) Date of filing: 13.10.2014
(51) Int. Cl.: A01N 33/12, A61K 9/00, A61K 47/10, A61K 9/08, A61K 49/00, A61K 31/14, A61K 31/785, A01N 47/44, A61K 31/155

(54) **BLUE DYED CHLORHEXIDINE ANTIMICROBIAL COMPOSITION FOR SKIN DISINFECTION**
BLAU GEFÄRBTE ANTIMIKROBIELLE CHLORHEXIDINZUSAMMENSETZUNG ZUR HAUTDESINFEKTION
COMPOSITION ANTIMICROBIENNE DE CHLORHEXIDINE À TEINTE BLEUE POUR DÉSINFECTION DE LA PEAU

(43) Date of publication of application: 23.08.2017
(73) Proprietor: Nex Medical Antiseptics S.r.l., 20010 Casorezzo (Milano) (IT)
(72) Inventor: DANELUZZI, Silvio, I-20010 Casorezzo (Milano) (IT); BIGNOZZI, Carlo, I-44121 Ferrara (IT)
(74) Representative: Ferreccio, Rinaldo
(86) International application number: PCT/IT2014/000268
(87) International publication number: WO 2016/059653

(56) References cited:
- EP-A1- 2 499 914
- CN-A- 103 356 738
- DE-A1- 4 137 548
- GB-A- 2 337 521
- US-A1- 2007 253 909
- "Chlorhexidine Irrigation Solution CONSUMER MEDICINE INFORMATION Before you use CHLORHEXIDINE IRRIGATION SOLUTION", , 25 January 2011 (2011-01-25), XP055178796, Retrieved from the Internet: URL:http://www.betterhealth.vic.gov.au/bhc v2/bhcmed.nsf/pages/pfcchlri/$File/pfcchlr i.pdf [retrieved on 2015-03-24]
- "SUMMARY OF PRODUCT CHARACTERISTICS 1. NAME OF THE VETERINARY MEDICINAL PRODUCT", , 1 April 2011 (2011-04-01), XP055178805, Retrieved from the Internet: URL:http://mri.medagencies.org/download/UK _V_0378_001_FinalSPC.pdf [retrieved on 2015-03-24]

## Description

The present invention relates, in general terms, to a blue dyed antimicrobial composition comprising chlorhexidine and a dye usable in the medical field. In particular, the invention relates to said composition as hydro-alcoholic solution and its use as a disinfectant and/or antiseptic agent, for example in the pre-surgical procedures.

### State of the art

Chlorhexidine is a chemical disinfectant endowed with antiseptic action, having the following formula:

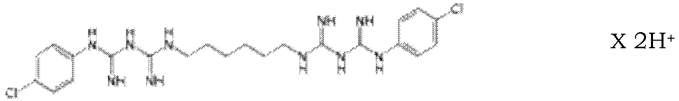

### (chlorhexidine)

Chlorhexidine is characterized by the fact of having a broad spectrum of action, being active towards Gram-positive bacteria and Gram-negative bacteria, as well as to fungi. Chlorhexidine has a bactericidal activity and acts by dramatically increasing the permeability of the bacterial cell membrane, thus altering the protein structure. This causes the precipitation of several cytoplasmic macromolecules, and the subsequent cell death, by lysis of the bacterial cell or the fungus.

The disinfectant solutions on the market are normally chlorhexidine digluconate compositions, where the double positive charge, localized on the protonated chlorhexidine, is balanced by the negative charges supported by two anions gluconate: (HO)CH₂⁻[CH₂ (OH)]₄-COO⁻. This fact is related to the high solubility of the salt which is much greater than the corresponding forms containing anions such as acetate or chloride.

Various combinations of chlorhexidine with other active ingredients are also present on the market, in order to enhance the antimicrobial action, such as: chlorhexidine + benzalkonium chloride; chlorhexidine + cetrimide; chlorhexidine + didecyldimethylammonium chloride; chlorhexidine + ethanol; chlorhexidine + hydroxyquinoline and chlorhexidine + sodium fluoride.

Chlorhexidine-based preparations are widely used in dentistry, urology, dermatology, gynecology, veterinary medicine and surgery procedures.

Alcoholic solutions of chlorhexidine 2% are, in particular, used in skin antisepsis and in the prevention of surgical site infections.

In preoperative procedures, chlorhexidine is sometimes associated with dyes, these latter mainly used in order to better detect and indicate the disinfected area which will then be subjected to a surgical treatment. In some invasive surgery, such as biopsies and the like, it is, in fact, required the use of coloring solutions or compositions that are able to visually identify the area to treat, also exploiting an antibacterial action as required by the surgery operation itself.

For this purpose, the chlorhexidine can be used in solutions comprising, for instance, the chlorhexidine digluconate salt and a red dye, such as the azorubine (also called E 122) to unequivocally visualize and identify the disinfected area, that will be the object of the surgery.

The use of sulfonated azo dyes such as Azorubine has, however, the disadvantage of causing the precipitation of chlorhexidine in hydro-alcoholic solutions, thus drastically reducing its antimicrobial activity. For this reason, it is often necessary to prepare the coloring antiseptic solution in the time period immediately prior to the use, so as to prevent the rapid precipitation (and thus deactivation) of the components. A similar deactivation also occurs when chlorhexidine is in contact with soaps or toothpaste containing the lauryl sulphate anion, which also causes the precipitation of the chlorhexidine cation.

On the other hand, the use of dyes containing anthocyanins, betalains or porphyrins does not allow to reach a color intensity suitable for an application in the medical field. Also, these dyes do not allow an appreciable marking of the skin, to such an extent to be usable in surgical procedures.

Therefore, there is still the need to obtain stable antimicrobial (disinfectants and antiseptics) solutions comprising the chlorhexidine cation and at least one dye having a high color intensity and high stability over the time, to be used, in particular, in the pre-surgical procedures.

The applicants have now surprisingly found that by using a composition comprising chlorhexidine and the dye 3,7-bis(Dimethylamino)-phenothiazin-5-ium chloride of formula (I), also known as Methylene Blue, it is possible to prepare colored and stable aqueous or hydro-alcoholic solutions, which maintain substantially unaltered the bactericidal power of chlorhexidine, and which can be conveniently used for the disinfection and identification of the patient local area, associated to the pre-surgical procedures.

The composition of the invention is particularly suitable, for example, for the disinfection of the point of insertion of venous or arterial catheters, for antisepsis of skin areas in the injection therapy, and in the preparation of the skin prior to invasive procedures, such as biopsies, downspouts, diagnostic punctures and surgeries.

### Summary of the Invention

In a first aspect, the invention relates to an antimicrobial coloring composition in the form of a hydro-alcoholic solution, comprising at least:
one lower C₁-C₄ alkyl alcohol in an amount between 60% and 70% w/w, chlorhexidine or a pharmaceutically acceptable salt thereof, and the dye 3,7-bis(dimethylamino)-phenothiazin-5-ium chloride of formula (I), in amounts between 0.04% w/w and 0.08% w/w 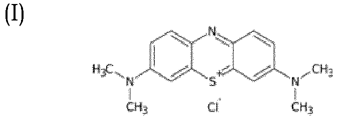
3,7-Bis(dimethylamino)-phenothiazin-5-ium chloride.

In a further aspect, the invention relates to said composition for use as a medicament, in particular as antimicrobial coloring agent in the pre-surgical procedures.

In a further aspect, the invention relates to said composition for use in a method for pre-surgical disinfection and identification of an area to be subjected to surgery, said method comprising the pre-surgical skin application of the hydro-alcoholic solution of the invention, in correspondence of the area of the human or animal body to be treated. This application can take place for example by contacting the affected skin area with the solution of the invention.

### Detailed Description

The term "% by weight" (% w/w) indicates the weight of the component with respect to the total weight.

The term "aqueous solution" includes in its meaning those solutions obtained by mixing the composition of the invention with water, and having an appearance substantially free from precipitates or solid residues.

Similarly, the term "hydro-alcoholic solution" includes in its meaning those solutions obtained by mixing the composition of the invention in a hydro-alcoholic solvent system comprising water and at least one lower C₁-C₄ alkyl alcohol, as described herein below in detail.

These hydro-alcoholic solutions are generally substantially free of solid residues, typical for example of a suspension.

For "hydro-alcoholic solvent system" is intended to indicate a liquid mixture comprising water and at least one lower C₁-C₄ alkyl alcohol, capable of dissolving the present composition, without leaving substantial precipitates or solid residues.

The term "lower C₁-C₄ alkyl alcohol " means a linear or branched alkyl alcohol compound having 1 to 4 carbon atoms, such as for example: ethanol, propanol, isopropanol and the like.

As above indicated, the present composition allows to obtain a medicament stable over the time, and widely usable in the medical field as coloring and antimicrobial agent.

In particular, the composition of the invention may be useful in the pre-surgical procedures for the detection and disinfection, preferably at skin level, of the body areas to be subjected to subsequent surgical treatment.

The present composition in the form of a hydro-alcoholic solution is characterized by the fact of having a high alcoholic percentage (in the order of 60-70% w/w) and a high chlorhexidine concentrations (which can be up to 4% p/p), keeping substantially unchanged over the time its disinfecting action and its color.

In one form of the invention, the chlorhexidine is present as a salt thereof, even more preferably in amounts between about 0.1% w/w and 4% w/w, preferably between about 1% w/w and 4% w/w, compared to the total weight.

Preferably, said salt is selected from: gluconate, digluconate, acetate and hydrochloride, being the digluconate (CAS No: 18472-51-0) particularly preferred.

The dye used in the present invention, i.e. the 3,7-bis(dimethylamino)-phenothiazin-5-ium chloride of formula (I) (CAS No. 61-73-4) is also known as Methylene Blue.

In one embodiment, the hydro-alcoholic solution comprises water and at least one lower C₁-C₄ alkyl alcohol selected from: ethyl alcohol and isopropyl alcohol.

Particularly preferred is a hydro-alcoholic solution comprising about 70% w/w of ethyl or isopropyl alcohol, and about 4% w/w of chlorhexidine digluconate. Surprisingly, applicants have noted that even in the presence of these high concentrations of active ingredients the final solutions are stable over the time and also in line with the current regulations provided by law (e.g. protocols CIPAC 46 and CIPAC 39.3), as shown in the below experimental part. Hydro-alcoholic solutions can be prepared by dissolving the composition of the invention in a suitable solvent system comprising water and at least one lower C₁-C₄ alkyl alcohol, as indicated above. Preferably, the solvent system of the invention comprises ionized, distilled, by injection or, even more preferably, osmotic water. Due to the high solubility of the components, the hydro-alcoholic solution of the invention can be obtained in a short frame of time by the addition to the chosen solvent system of the individual components (salt of chlorhexidine and dye (I), for example in the form of a homogeneous mixture, or singly. Hydro-alcoholic solutions including isopropyl alcohol or ethyl alcohol, and water are preferred ones.

The thus obtained solutions present a high degree of stability, being able to retain substantially unaltered the antimicrobial (antiseptic and disinfectant) activity of the chlorhexidine, being also able to perform the coloring function on the skin, due to the presence of the dye of formula (I).

Advantageously, therefore, the present invention allows the preparation and the storage for several months (or even years) of said solutions, avoiding in this way the preparation immediately prior to the surgical intervention, as it happens in the prior art in case of similar disinfecting and coloring compositions.

In a further embodiment, the present composition in the form of hydro-alcoholic solution can also include additional active ingredients having antimicrobial activity, preferably the didecyldimethylammonium chloride and/or benzalkonium chloride or polyhexamethylen biguanide.

In any case, either if the composition of the invention only contains the active principle chlorhexidine, or in the case where the chlorhexidine is in admixture with another antimicrobial agent, it is possible to obtain final solutions which are stable in time, and that, advantageously, allow an effective antibacterial and/or antiseptic action, even further enhanced by the combination of two or more active ingredients. Thanks to the possibility of a cutaneous administration, the present composition not only allow to visually indicate the site of the human or animal body which has to be subjected to surgery (thanks to the presence of the dye of formula (I)), but also it makes possible to exploit a wide-range antimicrobial activity, especially useful in the case of invasive transcutaneous interventions such as injections, biopsies or the like. Therefore, in an additional aspect, the invention relates to the present composition in the form of a hydro-alcoholic solution, for use as a medicament with coloring and antimicrobial action. In one embodiment, the invention relates to the above-described composition for use as a coloring antimicrobial agent for cutaneous administration.

This administration can be done either on an animal or on human body, usually by rubbing, dripping or similar procedures.

The present invention will now be described with the following experimental part without however limiting the scope.

### EXPERIMENTAL PART

The following hydro-alcoholic solutions gels were prepared and tested with regards to their stability.

### Example 1: preparation of a hydro-alcoholic solution comprising: Chlorhexidine 2%, Blue dye of formula (I) and Ethanol.

Composition: Chlorhexidine digluconate 2% w/w, Ethyl Alcohol (95%) 70% w/w, 3,7-bis(Dimethylamino)-phenothiazin-5-ium chloride (Methylene Blue) 0.08% w/w.

General procedure: 100 grams of Chlorhexidine digluconate (20%) were added with stirring to 200 grams of distilled water. Then 700 g of Ethyl Alcohol (95%) and 0.8 g of Methylene Blue were added thereto. Mixing for 10 min and packaging.

### Example 2: preparation of a hydro-alcoholic solution comprising: Chlorhexidine 2%, Blue dye of formula (I) and Iso-Propanol.

Composition: Chlorhexidine digluconate 2% Isopropyl Alcohol (99%) 70% w/w, 3,7-bis(Dimethylamino)-phenothiazin-5-ium chloride (Methylene Blue) 0.08% w/w.

General procedure: 100 grams of Chlorhexidine digluconate (20%) were added with stirring to 200 grams of distilled water. Then 700 g of iso-propylic alcohol (95%) and 0.8 g of Methylene Blue were added thereto. Mixing for 10 min and packaging.

### Example 3: preparation of a hydroalcoholic solution comprising: Chlorhexidine 4%, Blue dye of formula (I) and Ethanol.

Composition: Chlorhexidine digluconate 4%, Ethyl Alcohol (95%) 70%, 3,7-bis(Dimethylamino)-phenothiazin-5-ium chloride (Methylene Blue) 0.08% w/w.

General procedure: 200 g of Chlorhexidine digluconate (20%) were added under stirring to 100 grams of distilled water. Then 700 g of Ethyl Alcohol (95%) and 0.8 g of methylene blue were added thereto. Mixing for 10 min and packaging.

### Stability test of the compositions obtained in the Examples 1-3.

In all the cases of the above examples 1-6, the accelerated stability was evaluated according to standard protocols that encompasses the maintenance of the solutions of the Examples 1-3 at a temperature of about 54 ± 2° C, for a period of time equal to 14 days (study protocol CIPAC 46), and the maintenance of the same at 0 ± 2° C for a period of time of 7 days (study Protocol CIPAC 39.3).

Sample analysis includes both the initial and final control of concentrations of the various components, and the observation of the presence of stratification/precipitates.

All the samples of the Examples 1-3 gave positive results to the protocols of accelerated stability.

Worth to note that the obtained results of accelerated testing protocols provided by the CIPAC 46 and CIPAC 39.3, are indicative of a stability of the compositions corresponding to a period of time of 2 years.

## Claims

1. An antiseptic coloring composition, in the form of a hydro-alcoholic solution comprising at least one lower C₁-C₄ alkyl alcohol in an amount between 60% w/w and 70% w/w, at least chlorhexidine or a pharmaceutically acceptable salt thereof, and the dye 3,7-bis(dimethylamino)-phenothiazin-5-ium chloride of formula (I), in amounts between 0.04 w/w and 0.08% w/w.

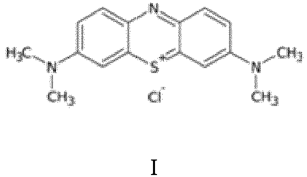

2. The composition according to claim 1, comprising a chlorhexidine salt selected from: gluconate, digluconate, acetate and hydrochloride.

3. The composition according to claim 2, wherein said chlorhexidine salt is the digluconate.

4. The composition according to the preceding claims, comprising chlorhexidine or a pharmaceutically acceptable salt thereof, in amounts between 0.1% w/w and 4% w/w.

5. The composition according to claim 4, comprising chlorhexidine or a pharmaceutically acceptable salt thereof in amounts between 1% w/w and 4% w/w.

6. The composition of claims 5, wherein said at least one lower C₁-C₄ alkyl alcohol, is selected from: ethanol and isopropanol.

7. The composition according to the preceding claims, comprising a lower C₁-C₄ alkyl alcohol 70% w/w, and chlorhexidine digluconate 4% w/w.

8. The composition according to the preceding claims, further comprising at least one compound with antimicrobial activity, preferably selected from: didecyldimethylammonium chloride, benzalkonium chloride, polyhexamethylene biguanide and mixtures thereof.

9. The composition according to the preceding claims, comprising the lower C₁-C₄ alkyl alcohol and water in a ratio ranging between 60/40 and 70/30.

10. The composition according to the preceding claims, for use as a medicament.

11. The composition according to claim 10, for use as a coloring antimicrobial agent.

12. The composition according to claims 10-11 for use as a coloring antimicrobial agent for cutaneous administration.

13. The composition according to claims 10-12 for use for the disinfection and identification of the patient local area, associated to pre-surgical procedures.

14. The composition in the form of a hydro-alcoholic solution according to claim 1-9 for use in a method for pre-surgical disinfection and identification of an area to be subjected to surgery, said method comprising the pre-surgical skin application of said hydro-alcoholic solution in correspondence of the area of the human or animal body to be treated.

## Patentansprüche

1. Antiseptische Färbezusammensetzung in Form einer hydroalkoholischen Lösung, umfassend mindestens einen niederen C₁-C₄-Alkylalkohol in einer Menge zwischen 60% w/w und 70% w/w, mindestens Chlorhexidin oder ein pharmazeutisch annehmbares Salz hiervon, und den Farbstoff 3,7-Bis(dimethylamino)-phenothiazin-5-iumchlorid der Formel (I) in Mengen zwischen 0,04 w/w und 0,08% w/w

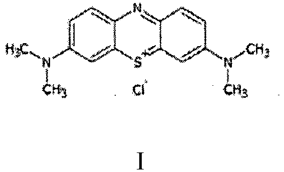

2. Zusammensetzung nach Anspruch 1, umfassend ein Chlorhexidinsalz, gewählt aus: Gluconat, Digluconat, Acetat und Hydrochlorid.

3. Zusammensetzung nach Anspruch 2, wobei das Chlorhexidinsalz das Digluconat ist.

4. Zusammensetzung nach den vorangehenden Ansprüchen, umfassend Chlorhexidin oder ein pharmazeutisch annehmbares Salz hiervon in Mengen zwischen 0,1% w/w und 4% w/w.

5. Zusammensetzung nach Anspruch 4, umfassend Chlorhexidin oder ein pharmazeutisch annehmbares Salz hiervon in Mengen zwischen 1% w/w und 4% w/w.

6. Zusammensetzung nach Anspruch 5, wobei der mindestens eine niedere C₁-C₄-Alkylalkohol gewählt ist aus: Ethanol und Isopropanol.

7. Zusammensetzung nach den vorangehenden Ansprüchen, umfassend einen niederen C₁-C₄-Alkylalkohol 70% w/w und Chlorhexidindigluconat 4% w/w.

8. Zusammensetzung nach den vorangehenden Ansprüchen, weiterhin umfassend mindestens eine Verbindung mit antimikrobieller Aktivität, vorzugsweise gewählt aus: Didecyldimethylammoniumchlorid, Benzalkoniumchlorid, Polyhexamethylenbiguanid und Mischungen hiervon.

9. Zusammensetzung nach den vorangehenden Ansprüchen, umfassend den niederen C₁-C₄-Alkylalkohol und Wasser in einem Verhältnis im Bereich zwischen 60/40 und 70/30.

10. Zusammensetzung nach den vorangehenden Ansprüchen zur Verwendung als ein Arzneimittel.

11. Zusammensetzung nach Anspruch 10 zur Verwendung als ein färbendes antimikrobielles Mittel.

12. Zusammensetzung nach den Ansprüchen 10-11 zur Verwendung als ein färbendes antimikrobielles Mittel für die kutane Verabreichung.

13. Zusammensetzung nach den Ansprüchen 10-12 zur Verwendung für die Desinfektion und Identifikation des Patienten-Lokalbereichs, verbunden mit präoperativen Verfahren.

14. Zusammensetzung in der Form einer hydroalkoholischen Lösung nach den Ansprüchen 1-9 zur Verwendung bei einem Verfahren zur präoperativen Desinfektion und Identifikation eines einer Operation zu unterziehenden Bereichs, wobei das Verfahren die präoperative Hautapplikation der hydroalkoholischen Lösung korrespondierend zu dem Bereich des zu behandelnden menschlichen oder tierischen Körpers umfasst.

## Revendications

1. Composition colorante antiseptique, sous la forme d'une solution hydroalcoolique comprenant au moins un alcool d'alkyle en C₁ à C₄ inférieur en une quantité comprise entre 60 % en poids/poids et 70 % en poids/poids, au moins de la chlorhexidine ou un sel pharmaceutiquement acceptable de celle-ci, et le colorant chlorure de 3,7-bis-(diméthylamino)-phénothiazin-5-ium de formule (**I**), en des quantités comprises entre 0,04 en poids/poids et 0,08 % en poids/poids.

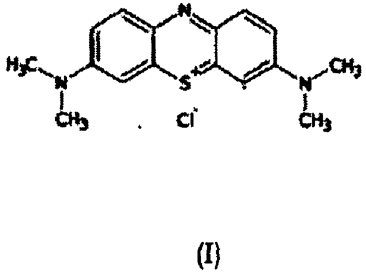

2. Composition selon la revendication 1, comprenant un sel de chlorhexidine sélectionné parmi : un gluconate, un digluconate, un acétate et un chlorhydrate.

3. Composition selon la revendication 2, dans laquelle ledit sel de chlorhexidine est le digluconate.

4. Composition selon les revendications précédentes, comprenant de la chlorhexidine ou un sel pharmaceutiquement acceptable de celle-ci, en des quantités comprises entre 0,1 % en poids/poids et 4 % en poids/poids.

5. Composition selon la revendication 4, comprenant de la chlorhexidine ou un sel pharmaceutiquement acceptable de celle-ci en des quantités comprises entre 1 % en poids/poids et 4 % en poids/poids.

6. Composition selon la revendication 5, dans laquelle ledit au moins un alcool d'alkyle en C₁ à C₄ inférieur, est sélectionné parmi : l'éthanol et l'isopropanol.

7. Composition selon les revendications précédentes, comprenant 70 % en poids/poids d'un alcool d'alkyle en C₁ à C₄ inférieur, et 4 % en poids/poids de digluconate de chlorhexidine.

8. Composition selon les revendications précédentes, comprenant en outre au moins un composé ayant une activité antimicrobienne, de préférence sélectionné parmi : le chlorure de didécyldiméthylammonium, le chlorure de benzalkonium, le polyhexaméthylène biguanide et les mélanges de ceux-ci.

9. Composition selon les revendications précédentes, comprenant l'alcool d'alkyle en C₁ à C₄ inférieur et de l'eau dans un rapport compris entre 60/40 et 70/30.

10. Composition selon les revendications précédentes, pour son utilisation en tant que médicament.

11. Composition selon la revendication 10, pour son utilisation en tant qu'agent antimicrobien colorant.

12. Composition selon les revendications 10 et 11 pour son utilisation en tant qu'agent antimicrobien colorant pour une administration cutanée.

13. Composition selon les revendications 10 à 12 pour son utilisation pour la désinfection et l'identification de la zone locale d'un patient, associée à des procédures pré-chirurgicales.

14. Composition sous la forme d'une solution hydroalcoolique selon les revendications 1 à 9 pour son utilisation dans un procédé de désinfection pré-chirurgicale et d'identification d'une zone à soumettre à une intervention chirurgicale, ledit procédé comprenant l'application sur une peau avant une intervention chirurgicale de ladite solution hydroalcoolique en correspondance avec la zone du corps d'un humain ou d'un animal à traiter.
